(19) Europäisches Patentamt
European Patent Office
Office européen des brevets

(11) **EP 0 603 962 B1**

(12) **EUROPEAN PATENT SPECIFICATION**

(45) Date of publication and mention
of the grant of the patent:
**17.02.1999 Bulletin 1999/07**

(51) Int Cl.⁶: **G01N 33/543**, G01N 33/78,
G01N 33/94

(21) Application number: **93203577.7**

(22) Date of filing: **17.12.1993**

(54) **Immunoassay elements having a separate receptor layer**

Immunoassay-Elemente, die eine getrennte Rezeptorschicht haben

Eléments d'essais immunologiques à couche de récepteurs séparées

(84) Designated Contracting States:
**AT BE CH DE FR GB IT LI NL SE**

(30) Priority: **22.12.1992 US 994857**

(43) Date of publication of application:
**29.06.1994 Bulletin 1994/26**

(73) Proprietor: **Johnson & Johnson Clinical
Diagnostics, Inc.
Rochester New York 14650 (US)**

(72) Inventors:
• **Sutton, Richard Calvin, c/o EASTMAN KODAK
COMPANY
Rochester, New York 14650-2201 (US)**
• **Mauck, Linda Ann, c/o EASTMAN KODAK
COMPANY
Rochester, New York 14650-2201 (US)**
• **Bowman, Wayne Arthur, c/o EASTMAN KODAK
COMPANY
Rochester, New York 14650-2201 (US)**

(74) Representative: **Mercer, Christopher Paul et al
Carpmaels & Ransford
43, Bloomsbury Square
London WC1A 2RA (GB)**

(56) References cited:
EP-A- 0 430 371      EP-A- 0 517 338
US-A- 5 059 526

## Description

This invention relates to an immunoassay element.

Immunoassays, which take advantage of natural immunological reactions, have found wide-spread use as analytical techniques in clinical chemistry. Because of the specificity of the reactions, they are particularly advantageous in quantifying biological analytes that are present in very low concentration in biological fluids. Such analytes include, for example, antigens, antibodies, therapeutic drugs, narcotics, enzymes, hormones, proteins, and so forth.

The analyte, which is the target of the assay is referred to herein as the ligand, and the labeled analyte is referred to as the labeled ligand (including immunocompetent derivatives and analogs of such ligand). Compounds which specifically recognize the ligand and the labeled ligand and react to form complexes with them are referred to herein as receptors. The receptor and the ligand or labeled ligand form a conjugate pair. Any member of the pair can function as a receptor or a ligand.

In competitive binding immunoassays, a labeled ligand is placed in competition with unlabeled ligand for reaction with a fixed amount of the appropriate receptor. Unknown concentrations of the ligand can be determined from the measured signal of either the bound or unbound (that is, free) labeled ligand. The reaction proceeds as follows:

$$\text{ligand} + \text{labeled ligand} + \text{receptor} <=>$$

$$\text{ligand-receptor} + \text{labeled ligand-receptor.}$$

Immunoassay analytical elements are known. In general such elements comprise receptors, such as antibodies for a ligand, immobilized in a particulate layer. In addition the element usually contains a reagent system that through interaction with a bound or unbound species results in a signal that can be correlated to the concentration of ligand in a sample. In use the sample is manually combined with an enzyme labeled ligand and applied to the element. After a time a solution containing a substrate for the labeled ligand is applied to the particulate layer. The reaction with substrate is catalyzed by the enzyme label to form a reaction product that ultimately causes a signal color to develop. The reflection density of the color can be correlated to the concentration of the ligand in the sample. Similar signal development systems are known for other known conventional labels such as radioactive tags, chromophores, fluorophores, stable free radicals, and enzyme cofactors, inhibitors and allosteric effectors.

Frequently the above analytical elements are used in highly automated immunoassays. The need to add labeled ligand to the sample before conducting the assay reduces the potential output of automated systems.

U.S. Patents 4,517,288 and 4,258,001 and EPA 0238012 suggest use of a labeled ligand coated over the particulate layer in which the receptor is immobilized. U.S. Patent 4,517,288 teaches using a barrier layer between the labeled ligand layer and the particulate layer in order to avoid prereaction between the receptor and the labeled ligand. EPA 0238012 suggests that a laminate of the two layers should be prepared under dried conditions and stored as a dry sheet until the commencement of the assay to avoid prebinding.

The problem is that neither of these patent publications teaches one skilled in the art how to make an immunoassay element that avoids (a) inactivation (low sensitivity) of receptors, such as antibodies, and (b) deleterious prebinding between the labeled ligand and the receptor which prevents completion of an immunoassay within a commercially reasonable time (up to 10 minutes).

EP-A-0517338 and EP-A-0430371 each attempts to provide a solution to this problem. EP-A-0517338 discloses a dry immunoassay element comprising a coating containing an enzyme labeled ligand directly over a particulate layer containing a fixed concentration of immobilized receptor without an intervening barrier layer. EP-A-0430371 discloses an analytical element for immunoassays comprising a support and a spreading layer, the element containing a first population of relatively large polymeric beads having a diameter in the range of 10 to 200 μm to facilitate uniform spreading of liquids applied to the element, and a second population of relatively small polymeric beads having a diameter in the range of 0.1 to 5 μm, receptors covalently bound to the bead surface through surface reactive groups and surfaces which are free of residual materials.

The objective of the present invention is to overcome the need to add labeled ligand to the sample or element during the actual assay procedure without adversely affecting the capacity of the receptor to be useful in an immunoassay element. This objective is accomplished by providing a dry immunoassay analytical element according to claim 1 and the claims dependent thereon. The element is substantially free of prebinding reactions between the receptor and the labeled ligand as defined below. The receptor exhibits good sensitivity compared to other elements having different receptor polymeric binders. Many binders tested were not operative. Coating without binder also resulted in an inoperative element. Therefore binders recited in the receptor layer are essential to providing an operative immunoassay element. The binders do not adversely affect the binding activity of antibody receptors.

Further the recited binders make it possible to form uniform coatings of receptor layers due to the very low binder viscosities achieved from sheer thinning during extrusion hopper coating. A further advantage is achieved with the recited binders in that, immediately after forming uniform coatings, the viscosity of the binders increases substantially resulting in a "set layer" that remains stable and uniform during wet transport and drying of the binders.

The immunoassay element of this invention is substantially free of prebinding in that the rate of change of transmission density (Dt) of an immunoassay carried out on such an element is substantially the same, including the polymeric binder in the receptor layer, as the rate of change of transmission density (Dt) obtained with the same element except that the labeled ligand is not coated in the element. Instead the labeled ligand is applied to the element, concurrently with the sample to be tested.

The present invention can be used in a method for the assay of immunologically reactive ligand in an aqueous liquid sample, comprising the steps of:

A. providing a dry immunoassay analytical element according to the present invention;
B. contacting a finite area of the layer comprising the enzyme labeled ligand with a sample of the liquid sample thereby forming (i) an immobilized ligand-receptor complex and (ii) an immobilized enzyme labeled ligand-receptor complex;
C. contacting the finite area of the layer comprising the enzyme labeled ligand with a substrate solution thereby catalyzing the development of a color; and
D. determining the concentration of the ligand colorimetrically.

The present invention can be made by a method that eliminates contact between a coated labeled ligand and a receptor for the ligand before a sample is applied to the element, comprising the steps of:

A. coating a receptor layer having the composition described above on a support ;
B. coating a spreading layer over the receptor layer; and
C. coating a layer containing an enzyme labeled ligand over the spreading layer; wherein each layer is allowed to dry before being overcoated.

The elements of this invention comprise a coating of a labeled ligand over a spreading layer. The spreading layer is coated over the receptor layer. The element can include additional layers such as those described infra. All of such layers, except the labeled ligand coating, can be coated using coating techniques known in this art and which are briefly described infra.

Preferably the labeled ligand is gravure coated to 1) minimize wet coverage of the labeled ligand coating composition, to avoid precontact of the labeled ligand with the receptor, while at the same time maintaining enough wetness to achieve uniform coverage of the labeled ligand and 2) achieve rapid drying in a way that a) removes substantially all of the coating solvent; b) avoids adversely affecting the porosity of the spread layer and spreading time and c) maintains sufficient enzyme activity.

The relative affinity of antibody and labeled ligand for each other is also an important factor in minimizing prebinding. This factor is controlled, as is well known by those skilled in this art, by manipulating the structure of the labeled ligand together with a prudent choice of antibody.

In general the level of coated labeled ligand coverage needed in an element is determined empirically for each specific immunoassay according to the following procedure:

1. Determine the concentration of labeled ligand needed to achieve acceptable immunoassay performance when the immunoassay is performed by contacting the analytical element with the labeled ligand concurrently with a sample. Acceptable assay performance is achieved when (a) the assay can be carried out in less than 20 minutes; (b) the dynamic range of the assay is such that the minimum and maximum ligand concentrations detectable cover a clinically useful concentration range; and (c) clinically significant ligand concentrations can be detected across the dynamic range.
2. Empirically determine the level of coated labeled ligand coverage needed with the same analytical element to achieve the above established acceptable assay performance by:

A. Coating, directly over the particulate receptor layer of the element used to establish optimum spotted labeled ligand levels, the labeled ligand at a coverage in g/m$^2$ that is some fraction, multiple or the same as the concentration of labeled ligand used in spotting the labeled ligand in 1, supra.
B. Conduct a series of assays with test samples containing a known concentration of the ligand.
C. Compare the results of the assays with the known concentration of ligand; and
D. Repeat steps B and C as needed, varying the labeled ligand coverage according to the results seen in step 2C to determine the labeled ligand coverage required.

Depending on the labeled ligand, the coverage of the labeled ligand could be less than, the same or several multiples greater (2X, 3X, 4X, and so forth) than the labeled ligand concentration needed when the same assay is carried out by spotting the labeled ligand directly on the analytical element.

Using the above guidelines, carefully controlled gravure coating procedures were successfully carried out using the following coverages and drying protocols. The labeled ligand coatings in the elements of the invention were prepared with a gravure machine (made by Yasui of Japan). Drying conditions used for all of the examples were 120°F (49°C) in the first drying section only. The second section was not used. The gravure cylinder used contained 295 cells/inch ($1.344 \times 10^{-8}$ cells/m$^2$). The cells had a depth of 19 microns, a width of 72 microns and a land width between cells of 12 microns. This cylinder will deliver 4.3 g/m$^2$ of coating composition containing the labeled ligand to the bead spreading layer using the direct gravure process at a coating machine speed of 50 ft/min (15.24 m/minute). Those skilled in the gravure coating arts will be readily able to adapt the previously described procedure to any gravure coating machine. The coating composition for the labeled ligand was as follows:

| Coated Labeled Ligand Coating Composition Based on 4.3 g/m$^2$ Wet Coverage | |
|---|---|
| Component | g/m$^2$ Dry Coverage |
| MOPS Buffer | .0045 |
| BSA (Bovine Serum Albumin) | .000215 |
| poly(acrylamide) | .00108 |
| 4'-Hydroxyacetanilide | .000325 |
| *Labeled ligand | .000016 |

*Labeled ligand has been coated anywhere between 4 and 64 mg/m$^2$

The remaining layers of the element can be coated using well known coating techniques in this art. However each layer is coated separately and allowed to dry before application of subsequent layers.

The spreading layer is porous and coated over the receptor layer. Materials for use in such layers are well known in the art of making dry analytical elements as disclosed, for example, in U.S. Patent 4,258,001. Such layers include macroporous layers made from cloth, paper, and so forth. A preferred particulate layer is a bead spreading layer (BSL). This layer can be easily constructed to have suitable porosity for use in the elements of the present invention to accommodate a test sample (for example, 1 to 100 mL), diluted or undiluted. Preferably, the spreading layer is isotropically porous, which property is created by interconnected spaces between the particles comprising the zone. By isotropically porous is meant that the spreading layer uniformly spreads the applied fluid radially throughout the layer.

Useful spreading layers, including bead spreading layers are disclosed in U.S. Patents 4,670,381; 4,258,001 and 4,430,436. Particularly useful spreading layers are those having a particulate structure formed by organo-polymeric particles and a polymeric adhesive for those particles described in U.S. Patent 4,258,001. The organo-polymeric particles useful in the spreading layer are generally heat-stable, spherical beads having a particle size in the range of from 10 to 40 mm in diameter or even smaller.

The particles can be composed of a wide variety of organic polymers, including both natural and synthetic polymers, having the requisite properties. Preferably, however, they are composed of one or more addition polymers described in the aforementioned patents.

The receptor layer is prepared and coated over a support. The receptors are covalently bonded to polymer particles through surface reactive groups on the receptor (nucleophilic free amino groups and sulfhydryl groups).

A general procedure for attaching receptors to the small polymer beads includes covalently attaching the selected receptor to the beads using generally known reactions. With many pendant groups for example the haloalkyl, 2-substituted activated ethylsulfonyl and vinylsulfonyl, the receptor can be directly attached to the beads. Generally, the beads are mixed with the receptor in an aqueous buffered solution (pH generally from 5 to 10) and a concentration of from 0.1 to 40 weight percent polymer particles (preferably from 0.1 to 10 weight percent). The amount of receptor is at a ratio to polymer of from 0.1:1000 to 1:10, and preferably from 1:100 to 1:10. Mixing is carried out at a temperature in the range of from 5 to 50°C, and preferably at from 5 to 40°C, for from 0.5 to 48 hours. Any suitable buffer can be used.

In some instances, the pendant reactive groups on the outer surface must be modified or activated in order to cause covalent attachment of the ligand. For example, carboxyl groups must be activated using known carbodiimide or carbamoylonium chemistry, described in EP 308235 published 22 July 1992 and U.S. Patent 5,155,166.

The attachment of the receptor to carboxyl group-containing monodispersed polymer beads, however, is carried out in two steps, the first of which involves contacting an aqueous suspension of the particles with a carbodiimide or a carbamoylonium compound to produce reactive intermediate polymer particles having intermediate reactive groups in place of the carboxyl groups. This step is carried out at a suitable pH using suitable acids or buffers to provide the desired pH. Generally, the pH is less than 6, but this is not critical as long as the reaction can proceed. More likely, the pH is between 3.5 and 7. The molar ratio of carbodiimide or carbamoylonium compound to the carboxyl groups on the surface of the particles is from 10:1 to 500:1.

In the second step of the method, the reactive intermediate formed in the first step is contacted with a reactive amine- or sulfhydryl-group containing receptor. A covalent linkage is thereby formed between the particles and the receptor. The weight ratio of the receptor to the polymeric particles is generally from 1:1000 to 1:1, and preferably from 1:100 to 1:10.

In other instances, an epoxy group on the outer surface can be hydrolyzed to form a diol compound capable of reacting with cyanogen bromide which can act as a coupling agent for amine groups in the immunological species. Aldehydes can react directly with amines to form a Schiff's base which can be subsequently reduced to form a covalent link. Alternatively, the aldehyde can be oxidized to an acid and chemistry identified above for carboxyl groups can be used to form an amide linkage.

Any reactive amine- or sulfhydryl-containing receptor can be attached to the monodispersed polymeric beads as long as that receptor contains a reactive amine or sulfhydryl group, respectively which will react with the reactive groups on the polymer or with the intermediate formed by the reaction of a carbodiimide or a carbamoylonium compound with carboxyl groups on the particles in the case in which the polymer has reactive carboxyl groups.

The small polymer beads having reactive groups that readily react directly with the amine or sulfhydryl groups on the receptors are simply mixed with the receptors, in an appropriate buffer if necessary, and allowed to react.

Polymers from which beads for the receptor can be selected include the following: poly($\underline{m}$ & $\underline{p}$-chloromethylstyrene), poly(styrene-$\underline{co}$-$\underline{m}$ & $\underline{p}$-chloromethylstyrene-$\underline{co}$-2-hydroxyethyl acrylate) (67:30:3 molar ratio), poly(styrene-$\underline{co}$-$\underline{m}$ & $\underline{p}$-chloroethylsulfonylmethylstyrene) (95.5:4.5 molar ratio), poly{styrene-$\underline{co}$-N-[$\underline{m}$ & $\underline{p}$-(2-chloroethylsulfonylmethyl)phenyl]acrylamide} (99.3:0.7 molar ratio), poly($\underline{m}$ & $\underline{p}$-chloromethylstyrene-$\underline{co}$-methacrylic acid) (95:5, 98:2 and 99.8:0.2 molar ratio), poly(styrene-co-m & p-chloroethylsulfonylmethylstyrene-co-methacrylic acid)(93.5:4.5:2 molar ratio), poly{styrene-$\underline{co}$-N-[$\underline{m}$ & $\underline{p}$-(2-chloroethylsulfonylmethyl)phenyl]acrylamide-$\underline{co}$-methacrylic acid}(97.3:0.7:2 molar ratio), poly(styrene-$\underline{co}$-$\underline{m}$ & $\underline{p}$-chloromethylstyrene) (70:30 molar ratio), poly[styreneco-3-(p-vinylbenzylthio)propionic acid] (97.6/2.4 molar ratio), poly(styrene-$\underline{co}$-vinylbenzyl chloride-$\underline{co}$-acrylic acid) (85:10:5 molar ratio), poly(styrene-$\underline{co}$-acrylic acid) (99:1 molar ratio), poly(styrene-$\underline{co}$-methacrylic acid) (90:10 molar ratio), poly(styrene-$\underline{co}$-acrylic acid-$\underline{co}$-$\underline{m}$ & $\underline{p}$-divinylbenzene) (89:10:1 molar ratio), poly(styrene-$\underline{co}$-2-carboxyethyl acrylate) (90:10 molar ratio), poly(methyl methacrylate-$\underline{co}$-acrylic acid) (70:30 molar ratio), poly(styrene-$\underline{co}$-$\underline{m}$ & $\underline{p}$-vinylbenzaldehyde) (95:5 molar ratio), and poly(styreneco-$\underline{m}$ & $\underline{p}$-vinylbenzaldehyde-$\underline{co}$-methacrylic acid) (93:5:2 molar ratio).

The element is carried on a suitable support. The receptor layer is coated over the support although there may be intervening layers, such as a gelatin/buffer layer, between the support and the receptor layer. The support can be any suitable dimensionally stable, and preferably, nonporous and transparent (that is, radiation transmissive) material which transmits electromagnetic radiation of a wavelength between 200 and 900 nm. A support of choice for a particular element should be compatible with the intended mode of detection (reflection, transmission or fluorescence spectroscopy). Useful support materials include polystyrene, polyesters [for example, poly(ethylene terephthalate)], polycarbonates, cellulose esters (for example, cellulose acetate), and so forth.

The required polymeric binders for the receptor layer are described generally in Canadian patent 1,240,445 and are expressly incorporated herein by reference. Useful polymers are chill-gelable polymers comprising from 30 to 97 weight percent of polymerized N-alkyl substituted acrylamide such as N-isopropylacrylamide. Other useful N-alkyl-substituted acrylamides include N-$\underline{n}$-butylacrylamide, N,N-diethylacrylamide and N-$\underline{n}$-propylacrylamide. Polymer binders comprising 60 to 97 weight percent of polymerized N-isopropylacrylamide are used in the examples to clarify the utility of these binders.

The polymer binder also comprises from 3 to 25 weight percent of one or more polymerized crosslinking monomers having at least two addition-polymerizable groups per molecule. These crosslinking monomers are generally well known in the art. The preferred crosslinking monomers contain acrylamido or methacrylamido groups to facilitate polymerization with the N-alkyl-substituted acrylamides.

Examples of useful crosslinking monomers include:

N,N'-methylenebisacrylamide;
N,N'-methylenebismethacrylamide;
ethylene dimethacrylate;
2,2-dimethyl-1,3-propylene diacrylate;
divinylbenzene;
mono[2,3-bis(methacryloyloxy)propyl] phosphate;
N,N'-bis(methacryloyl)urea;
triallyl cyanurate;
allyl acrylate;

allyl methacrylate;

N-allylmethacrylamide;

4,4'-isopropylidenediphenylene diacrylate;

1,3-butylene diacrylate;

1,4-cyclohexylenedimethylene dimethacrylate;

2,2'-oxydiethylene dimethacrylate;

divinyloxymethane;

ethylene diacrylate;

ethylidene diacrylate;

propylidene dimethacrylate;

1,6-diacrylamidohexane;

1,6-hexamethylene diacrylate;

1,6-hexamethylene dimethacrylate;

phenylethylene dimethacrylate;

tetramethylene dimethacrylate;

2,2,2-trichloroethylidene dimethacrylate;

ethylenebis(oxyethylene) diacrylate;

ethylenebis(oxyethylene) dimethacrylate;

ethylidyne trimethacrylate;

propylidyne triacrylate;

vinyl allyloxyacetate;

1-vinyloxy-2-allyloxyethane;

2-crotonoyloxyethyl methacrylate;

diallyl phthalate; and

2-(5-phenyl-2,4-pentadienoyloxy) ethyl methacrylate.

The receptor polymeric binder can include 0 to 60 weight percent of polymerized hydrophilic monomers. Amounts of 5 to 35 weight percent are also useful. Hydrophilic monomers are disclosed in Canadian patent 1,240,445. In particular such monomers have one or more groups selected from hydroxy, pyrrolidone, amine, amide, carboxy, sulfo, carboxylate salt, sulfonate salt and sulfate salt groups. Generally the counter ions of the salt groups are alkali metal or ammonium. Useful hydrophilic monomers are acrylic acid and methacrylic acid and their salts, 2-acrylamido-2-methylpropane sulfonate, 2-hydroxyethyl acrylate, 2-hydoxyethyl methacrylate, 2-hydroxypropyl acrylate, 2-hydroxypropyl methacrylate and glyceryl methacrylate.

The element can comprise one or more additional layers, for example, a separate or combined reagent/spreading layer and a gelatin/buffer layer containing other necessary additives such as electron transfer agents.

The gelatin/buffer layer or the reagent layer or the spreading layer of the element can contain the indicator composition comprising one or more reagents dispersed in one or more synthetic or natural binder materials, such as gelatin, or other naturally-occurring colloids, homopolymers and copolymers, such as poly(acrylamide), poly(vinylpyrrolidone), poly(N-isopropylacrylamide), poly(acrylamide-co-N-vinyl-2-pyrrolidone) and similar copolymers. The indicator composition can also be dispersed in the receptor layer.

Other optional layers, for example, subbing layers, radiation-blocking layers, and so forth can be included if desired. All layers of the element are in fluid contact with each other, meaning that fluids and reagents and uncomplexed reaction products in the fluids can pass between superposed regions of adjacent layers.

The layers of the element can contain a variety of other desirable but optional components, including surfactants, thickeners, buffers, hardeners, antioxidants, coupler solvents, and other materials known in the art. The amounts of these components are also within the skill of a worker in the art.

The elements can be used to determine low concentrations of immunologically reactive ligands in a liquid, such as a biological fluid (for example, whole blood, serum, plasma, urine, spinal fluid, suspensions of human or animal tissue, feces, saliva, lymphatic fluid and the like). The ligands can be determined at concentrations as low as $10^{-15}$ molar, and most generally at a concentration of from $10^{-11}$ to $10^{-4}$ molar.

Ligands which can be so determined, either quantitatively or qualitatively, include therapeutic drugs (for example, phenobarbital, digoxin, digitoxin, theophylline, gentamicin, quinidine, phenytoin, propanolol, carbamazepine, tobramycin, lidocaine, procainamide and the like), natural or synthetic steroids (for example, cortisol, aldosterone, testosterone, progesterone, estriol, and so forth), hormones (for example, thyroid hormones, peptide hormones, insulin, etc.), proteins (for example, albumin, IgG, IgM, ferritin, blood clotting factors, C-reactive protein, isoenzymes, apolipoproteins, etc.), antigens, antibodies including monoclonal antibodies, and other species which will naturally react with a receptor. This invention is particularly useful for the determination of therapeutic drugs, such as digoxin, phenytoin, theophylline, or phenobarbital and hormones such as thyroxine or triiodothyronine.

The assay can be carried out using any enzyme label which can be attached to the ligand to form a labeled ligand. Enzymes, such as glucose oxidase, peroxidases such as horseradish peroxidase (HRP), alkaline phosphatase and galactosidase are preferred labels.

It is within the skill of the ordinary worker in clinical chemistry to determine a suitable substrate for a given label. The substrate can be a material which is directly acted upon by the enzyme label, or a material that is involved in a series of reactions which involve enzymatic reaction of the label. For example, if the enzyme label is a peroxidase, the substrate is hydrogen peroxide. Using glucose oxidase as an example, the substrate glucose is generally present in the reagent layer or added as a substrate solution to yield 0.01 moles/m$^2$, and preferably from 0.001 to 0.1 mole/m$^2$. A worker skilled in the art would know how to adjust the amount of a particular substrate for the amount of enzyme label used in the assay.

The reagent layer may contain an indicator composition comprising one or more reagents which provide a detectable species as a result of the reaction catalyzed by the label. The detectable species could develop a color, be radioactive, fluoresce, or be chemiluminescent. For present purposes the invention is illustrated using a colorimetric indicator composition which provides a colorimetrically detectable species as a result of enzymatic reaction of an enzyme-labeled ligand analog with a substrate.

The indicator composition can be a single compound which produces a detectable dye upon enzymatic reaction, or a combination of reagents which produce the dye. For example, when glucose is used as the substrate and glucose oxidase as the enzyme label, the colorimetric indicator composition can include a coupler and an oxidizable compound which react to provide a dye. Alternatively, the composition can include a leuco dye and peroxidase or another suitable peroxidative compound which generates a detectable dye as a result of the formation of hydrogen peroxide produced when glucose oxidase converts glucose to gluconic acid. Useful leuco dyes are known in the art and include those, for example, described in U.S. Patent 4,089,747 (issued May 16, 1978 to Bruschi) and U.S. Serial No. 612,509, filed May 21, 1984 by Babb and others. The particular amounts of the colorimetric indicator composition and its various components are within the skill of a worker in the art.

The labeled ligands can be prepared using known starting materials and procedures, or obtained commercially. Generally, the ligand is attached to the label (for example, an enzyme moiety) through a covalent bond.

The immunoassay can be manual or automated. In general, the amount of a ligand in a liquid is determined by taking the element from a supply roll, chip packet or other source and physically contacting a finite area of the spreading layer with a sample of the liquid, for example, 1 to 100 mL. The finite area which is contacted is generally no more than 150 mm$^2$.

The amount of ligand is determined by passing the element through a suitable apparatus for detecting the complexed ligand analog directly or the detectable species formed as a result of enzymatic reaction of an enzyme label and a substrate. For example, the species can be detected with suitable spectrophotometric apparatus using generally known procedures. In an enzymatic reaction, the resulting product is determined by measuring, for example, the rate of change of reflection or transmission density in the finite area which was contacted with the test sample. The area which is measured is generally from 3 to 5 mm. The amount of ligand in the liquid sample is inversely proportional to the amount of label measured in the finite area. Generally, label measurement is made after application of a substrate solution.

A typical element of this invention is presented below. It will be understood by those skilled in the art that the principle of the present invention can be usefully incorporated into any immunoassay element.

| Typical Immunoassay Element of the Invention | | |
|---|---|---|
| | | Dry Coverage (g/M$^2$) |
| Labeled Ligand Coating | digoxin-HRP | .000016 |
| | MOPS, pH 7.0 | .0045 |
| | Bovine serum albumin | .000215 |
| | Polyacrylamide | .00108 |
| | 4'-Hydroxyacetanilide | .000325 |

(continued)

| Typical Immunoassay Element of the Invention | | |
|---|---|---|
| | | Dry Coverage (g/M$^2$) |
| Bead Spreading Layer (BSL) | TES, pH 7.0 | .219 |
| | Dimedone | 0.5 |
| | Triarylimidazole leuco dye | .2 |
| | Dimethyl sulfoxide | 1.8 |
| | Poly(methyl acrylate-co-sodium 2-acrylamido-2-methylpropanesulfonate-co-2-acetoacetoxylethyl methacrylate) adhesive | 2.583 |
| | Bovine serum albumin | 0.5 |
| | Poly(m-& p vinyltoluene-co-methacrylic acid) Beads | 130 |
| | Zonyl FSN 4'-hydroxyacetanilide | .054 |
| Receptor Layer | Triton TX-100 | 0.02 |
| | TES.pH7.0 | 0.1 |
| | Antidigoxin beads (1 mm) | 0.015 |
| | binder | 1.0 |
| Gelatin Layer | gelatin | 10 |
| | 4'-Hydroxyacetanilide | .15 |
| | TES, pH 7.0 | 4.58 |
| | Triton TX-100 | .02 |
| | BVSME (hardener) | .15 |

The above element can be used to quantify ligands other than digoxin stated hereinbefore. The selected labeled ligand depends upon the particular assay. Only one such labeled ligand is used in a single assay with a paired antibody in the receptor layer so that a specific antibody-ligand pair exists for each assay.

The names and symbols used in the above element and the following examples have the following meanings:

| | |
|---|---|
| MOPS: | 3-Morpholinopropanesulfonic acid buffer |
| TES: | N[tris(hydroxymethyl)methyl]-2-aminoethanesulfonic acid |
| Dimedone: | 5,5-Dimethyl-1,3-cyclohexanedione Triarylimidazole leuco dye: 4,5-Bis(4-dimethyl-aminophenyl)-2-(4-hydroxy-3,5-dimethoxyphenyl)imidazole blue-forming leuco dye. |
| Magenta Dye: | 4,5-dihydroxy-3-(6,8-disulfo-2-napthylazo)-2,7-naphthalenedisulfonic acid, sodium salt |
| Zonyl FSN: | A nonionic, fluorinated surfactant sold by E. I. du Pont de Nemours. |
| Antidigoxin beads: | Poly[styrene-co-3-(p-vinylbenzylthio)propionic acid] molar ratio 87.6/2.4, weight ratio 95/5 having antidigoxin antibody covalently bound thereto. |
| PVA: | poly(vinyl alcohol) |
| TX-100: | Triton X-100, an octylphenoxy polyethoxy ethanol nonionic surfactant sold by Rohm and Haas. |
| HRP: | Horseradish peroxidase |
| BVSME: | Bis(vinylsulfonylmethyl) ether gelatin hardener. |
| PVP-K15: | Poly-N-vinylpyrrolidone with an average MW of 10,000. |
| PVP-K30: | same as PVP-K15 with an average MW of 40,000. |
| PVP-K90: | same as PVP-K15 with an average MW of 360,000. |
| BSA: | Bovine serum albumin. |

The invention is illustrated by the following examples:

Example 1

This examples demonstrates that the polymeric binder used in the receptor layer has minimum adverse affect on the binding activity of an antibody receptor.

Digoxin antibodies and horseradish peroxidase labeled digoxin (dgxn-hrp) are used to illustrate the invention.

Comparison assays were carried out on two identical elements having the same polymeric binder in the antibody receptor layer. The first element in the comparison had a structure according to the typical element presented hereinbefore except the labeled ligand, horseradish peroxidase-digoxin, was coated over the bead spread layer according to the present invention. The second element in the comparison was identical except it was free of the coated labeled ligand. The assay was carried out on the first element by simply spotting either 0 or 6 ng/mL of digoxin only. The assay was carried out on the second element by spotting the labeled ligand concurrently with either 0 or 6 ng/mL of digoxin. The elements were then incubated at 37 degrees for 5 minutes. The elements were then removed from the incubator and washed with 10mL of a wash fluid containing 0.03% hydrogen peroxide. Thereafter the elements were placed in an incubator at 37 degrees, and the rate of leuco dye oxidation was measured.

The difference in the rate measured by change in transmission density/minute (Dt per minute or delta rate) between the high and low concentration of digoxin was used as a measure of sensitivity. Dt is determined by measuring reflectance density which is converted to transmission using the well known Clapper-Williams transform. When the sensitivity for assays carried out on both elements is similar, no prebinding has occurred in the element of the invention.

The dry coverage of the receptor layer binder should be in the range of $0.2 g/m^2$ to $4.0 \ g/m^2$ to make receptor layers. The dry coverage should not be so great as to decrease sensitivity.

For further comparison and to show the effects of prebinding in the absence of a separate receptor layer Table 1 also includes the results obtained with analytical elements in which the antibody receptor is in the bead spreading layer (BSL). Note that the data shows significant prebinding with gravure coated labeled ligand.

Table 1

| Receptor Layer Binder | Coverage ($g/m^2$) | spotted or Gravure Coated Label | Rate $\Delta D_{t/min}$ |
|---|---|---|---|
| No receptor layer (antibody in the BSL) | -- | spotted<br>gravure | 0.049<br>0.037 |
| Poly(N-isopropylacrylamid e-co-methacrylic acid-co-N,N'-methylenebisacryla mide (weight ratio 80/10/10) | 1.0 | spotted<br>gravure | .030<br>.029 |

This comparisons show that with the receptor layer binder in Table 1 the element of the invention experienced no prebinding and the antibody receptors exhibited good binding activity.

Other polymer binders tested in the receptor layer according to the above tests provided surprisingly adverse affects on the antibody receptor. Gelatin destroyed the antibody binding capacity completely. With poly(methyl acrylate-co-sodium 2-acrylamido-2-methylpropanesulfonate-co-2-acetoacetoxyethyl methacrylate) at coverages of 1 or 1.5mg/$m^2$ the antibody exhibited a delta rate that was too low for use in the receptor layer of an immunoassay element. Poly (acrylamide) was also unacceptable. Coating without a binder resulted in antibody receptors having no binding capacity.

Example 2:

This example demonstrates the superior coating uniformity achieved with the receptor layer binders of the present invention.

A series of analytical elements was prepared by extrusion hopper coating wherein each layer of the element was dried before coating the next layer. Six elements were prepared using, as a control, poly-N-vinylpyrrolidone (average MW of 360,000.) as the receptor layer binder and 11 elements were prepared using a polymer of the invention, poly(N-isopropylacrylamide-co-methacrylic acid-co-N,N'-methylenebisacrylamide) (weight ratio 80/10/10), as the receptor layer binder. Higher molecular weight poly-N-vinylpyrrolidones (viscosity above 60 cps), were difficult to evenly extrude and the coatings were more nonuniform than the control.

A measure of "uniformity" is the "$\Delta D_t$ rate imprecision". The lower the standard deviation (sd) of the rate, the better the layer uniformity. The pooled standard deviation of the above-mentioned elements was determined as follows. Each of the receptor layers were assembled separately into the following dry analytical element.

| | | Dry Coverage (g/M$^2$) |
|---|---|---|
| Labeled Ligand Coating | digoxin-HRP | .000016 |
| | MOPS, pH 7.0 | .0045 |
| | Bovine serum albumin | .000215 |
| | Polyacrylamide | .00108 |
| | 4'-Hydroxyacetanillde | .000325 |
| | magenta dye | .0269 |
| Bead Spreading Layer (BSL) | TES, pH 7.0 | .219 |
| | Dimedone | 0.5 |
| | Triarylimidazole leuco dye | .2 |
| | Dimethyl sulfoxide | 1.8 |
| | Poly(methyl acrylate-co-sodium 2-acrylamido-2-methylpropanesulfonate-co-2-acetoacetoxylethyl methacrylate) adhesive | 2.583 |
| | Bovine serum albumin | 1.0 |
| | Poly(m-& p vinyltoluene-co-methacrylic acid) Beads (30m) | 130 |
| | glycerol | 2.0 |
| | 4'-hydroxyacetanilide | .45 |
| | mannitol | 1.0 |
| Receptor Layer | Triton TX-100 | 0.02 |
| | TES.pH 7.0 | 0.1 |
| | Antidigoxin beads (0.5 mm) | 0.015 |
| | receptor layer binder | 0.7 |
| Gelatin Layer | gelatin | 10 |
| | 4'-Hydroxyacetanilide | .15 |
| | TES, pH 7.0 | 4.58 |
| | Triton TX-100 | .02 |
| | BVSME (hardener) | .15 |

The magenta dye in the element is 4,5-dihydroxy-3-(6,8-disulfo-2-naphthylazo)-2,7-naphthalenedisulfonic acid, sodium salt.

Each element was spotted with 11 mL of either 0.05 ng/mL or 3.1 ng/mL digoxin in a human serum-based fluid, then placed in an incubator at 37°C for 5 minutes. The elements were then removed from the incubator and washed with 12 mL of a fluid containing hydrogen peroxide. The elements were placed back into the incubator, and the rate of color formation in the center of the slide was determined using reflectance densitometry as described in example 1. For each receptor layer, 10 elements were analyzed with each fluid. The individual rate sd's were pooled using conventional statistical methods and the statistical significance of the ratio of the variances tested using the F test described in *Statistics for Experimenters, An Introduction to Design, Data Analysis, and Model Building* by Hunter and others, John Wiley & Sons N.Y., N.Y. (1978).

| Receptor layer binder | g/m$^2$ | Melt Viscosity (low shear) | Pooled $\Delta D_{t/min}$ sd | No. of Coatings |
|---|---|---|---|---|
| Control | 1.0 | 30cps | 0.00307 | 6 |
| Invention polymer | 0.7 | * | 0.00192 | 11 |

*The polymers of the invention undergo shear thinning at lower shear rates than most polymer melts and many polymer solutions. The viscosity of a 2.5% solution of the ionic polymers of this invention at a pH 7 will drop from above 200 poise or more at 25°C to 20 poise or less as shear rate increases, with over 60%, generally over 90%, of the viscosity drop occurring at a shear rate less than $10^2$/sec. The nonionic polymers of the invention exhibit similar behavior at higher concentrations i.e. above 7%. Because of this viscosity change with changing shear rate, accurate viscosities are difficult to measure, and profiles of viscosity vs shear rate are generally provided instead. Most polymer melts and solutions do not exhibit shear thinning until the shear rate is above $10^2$/sec to $10^3$/sec.

The data show that the polymers of the invention have a much lower $\Delta D_t$ rate imprecision than the control and predictably most other polymers. Thus greater coating uniformity is achieved than with the control polymer.

After the uniformly coated layers have been formed high viscosities are exhibited immediately after coating. This results in a "set" layer that remains stable and uniform during wet transport and drying. Typically, a viscosity at zero shear of over 90 cps is desired to facilitate transport and drying of wet coated layers, while a viscosity of 10-80 cps or less is desired for uniform extrusion hopper coating. The polymers of the invention do not degrade the binding activity of the antibody.

## Claims

1.    A dry immunoassay analytical element, for assaying a ligand, comprising in the following order (a) a layer containing a labeled ligand, (b) a spreading layer and c) a receptor layer containing a fixed concentration of an immobilized receptor for the labeled ligand and d) a support; characterised in that the receptor is i) covalently bonded to polymeric beads having a diameter in the range of 0.1 to 5 mm and ii) dispersed in a binder of a crosslinked chill-gelable polymer comprising 30 to 97 weight percent of polymerized N-alkyl-substituted acrylamide monomers, 3 to 25 weight percent polymerized crosslinking monomer having at least two addition polymerizable groups per molecule, and 0 to 60 weight percent of other polymerized hydrophilic monomers.

2.    The element of claim 1 characterised in that the N-alkyl substituted acrylamide is present in an amount of 60 to 97 weight percent and is selected from N-isopropylacrylamide, N-n-butylacrylamide, N,N-diethylacrylamide and N-n-propylacrylamide.

3.    The element of claim 2 characterised in that the N-alkyl substituted acrylamide is N-isopropylacrylamide.

4.    The element of claim 2 or claim 3 characterised in that the crosslinking monomer is N,N'-methylenebisacrylamide.

5.    The element of claim 1 characterised in that the hydrophilic monomer is selected from methacrylic acid, 2-hydroxyethyl methacrylate, acrylic acid and 2-hydroxyethyl acrylate

6.    The element of claim 1 characterised in that the binder is poly(N-isopropylacrylamide-co-methacrylic acid-co-N,N'-methylenebisacrylamide).

7.    The element of any one of the preceding claims characterised in that the receptor is an antibody for the ligand, the label on the ligand is an enzyme and the labeled ligand is present at a coverage less than, the same as or some multiple of the amount of labeled ligand used when the assay is carried out by contacting the analytical element concurrently with a test sample and labeled ligand.

8.    The element of claim 7 characterised in that the labeled ligand is present above the spreading layer at a coverage of at least 4 to 64 mg/m2 .

9.    The element of claim 8 characterised in that the ligand comprises a hapten or hapten analog selected from digoxin, phenytoin, carbamazepine, phenobarbital, theophylline and thyroxine.

10.    The element of claim 9 characterised in that is has an additional layer containing an electron transfer agent and a buffer between the receptor layer and the support.

**Patentansprüche**

1. Trockenes, analytisches Immunoassay-Element zum Nachweis eines Liganden, das in der folgenden Reihenfolge (a) eine Schicht, welche einen markierten Liganden enthält, (b) eine Ausbreitungsschicht und (c) eine Rezeptorschicht, welche eine festgelegte Konzentration eines immobilisierten Rezeptors für den markierten Liganden enthält, und (d) einen Träger umfaßt, dadurch gekennzeichnet, daß der Rezeptor i) covalent an polymere Kügelchen mit einem Durchmesser im Bereich von 0,1 - 5 mm gebunden und ii) in einem Binder von vernetztem, Kältegelierbarem Polymer dispergiert ist, welches 30 - 97 Gewichtsprozent von polymerisierten, N-alkylsubstituierten Acrylamidmonomeren, 3 - 25 Gewichtsprozent polymerisiertes, vernetzendes Monomer mit mindestens zwei additionspolymerisierbaren Gruppen pro Molekül und 0 - 60 Gewichtsprozent an anderen polymerisierten, hydrophilen Monomeren umfaßt.

2. Element nach Anspruch 1, dadurch gekennzeichnet, daß das N-alkylsubstituierte Acrylamid in einer Menge von 60 - 97 Gewichtsprozent vorliegt und ausgewählt ist aus N-Isopropylacrylamid, N-n-Butylacrylamid, N,N-Diethylacrylamid und N-n-Propylacrylamid.

3. Element nach Anspruch 2, dadurch gekennzeichnet, daß das N-alkylsubstituierte Acrylamid N-Isopropylacrylamid ist.

4. Element nach Anspruch 2 oder 3, dadurch gekennzeichnet, daß das vernetzende Monomer N,N'-Methylenbisacrylamid ist.

5. Element nach Anspruch 1, dadurch gekennzeichnet, daß das hydrophile Monomer aus Methacrylsäure, 2-Hydroxyethyl-Methacrylat, Acrylsäure und 2-Hydroxyethyl-Acrylat ausgewählt ist.

6. Element nach Anspruch 1, dadurch gekennzeichnet, daß der Binder Poly(N-Isopropylacrylamid-co-methacrylsäure-co-N,N'-Methylenbisacrylamid) ist.

7. Element nach einem der vorangegangenen Ansprüche, dadurch gekennzeichnet, daß der Rezeptor ein Antikörper für den Liganden ist, die Markierung am Liganden ein Enzym ist, und der markierte Ligand in einer Beschichtungsmenge vorliegt, die weniger als, genau so viel wie oder ein Vielfaches der Menge an markiertem Liganden beträgt, welche verwendet wird, wenn der Assay durch gleichzeitiges In-Kontakt-Bringen des analytischen Elements mit einer Testprobe und markiertem Liganden durchgeführt wird.

8. Element nach Anspruch 7, dadurch gekennzeichnet, daß der markierte Ligand oberhalb der Ausbreitungsschicht in einer Beschichtungsmenge von mindestens 4 - 64 mg/m$^2$ vorliegt.

9. Element nach Anspruch 8, dadurch gekennzeichnet, daß der Ligand ein Hapten oder Hapten-Analog umfaßt, welches aus Digoxin, Phenytoin, Carbamazepin, Phenobarbital, Theophyllin und Thyroxin ausgewählt ist.

10. Element nach Anspruch 9, dadurch gekennzeichnet, daß es eine zusätzliche Schicht hat, welche ein Elektronentransfer-Agens und einen Puffer zwischen der Rezeptorschicht und dem Träger enthält.

**Revendications**

1. Elément analytique sec pour analyse immunologique, pour analyser un ligand, qui comprend dans cet ordre (a) une couche contenant un ligand marqué, (b) une couche d'étalement, et (c) une couche de récepteur contenant une concentration fixe d'un récepteur immobilisé du ligand marqué, et (d) un support ; caractérisé en ce que le récepteur est i) lié par liaison covalente à des perles polymères ayant un diamètre compris entre 0,1 et 5 mm, et ii) dispersé dans un liant d'un polymère réticulé pouvant gélifier à froid, comprenant de 30 à 97 % en poids d'acrylamides N-alkylés monomères polymérisés, de 3 à 25 % d'un monomère réticulable polymérisé ayant par molécule au moins deux groupes polymérisables par addition, et de 0 à 60 % en poids d'autres monomères hydrophiles polymérisés.

2. Elément selon la revendication 1, caractérisé en ce que l'acrylamide N-alkylé est présent en une quantité de 60 à 97 % en poids et est choisi parmi le N-isopropylacrylamide, le N-n-butylacrylamide, le N,N-diéthylacrylamide et le N-n-propylacrylamide.

3. Elément selon la revendication 2, caractérisé en ce que l'acrylamide N-alkylé est le N-isopropylacrylamide.

4. Elément selon la revendication 2 ou 3, caractérisé en ce que le monomère réticulable est le N,N'-méthylènebisacrylamide.

5. Elément selon la revendication 1, caractérisé en ce que le monomère hydrophile est choisi parmi l'acide méthacrylique, le méthacrylate de 2-hydroxyéthyle, l'acide acrylique et l'acrylate de 2-hydroxyéthyle.

6. Elément selon la revendication 1, caractérisé en ce que le liant est le poly(N-isopropylacrylamide-co-acide méthacrylique-co-N,N'-méthylènebisacrylamide).

7. Elément selon l'une quelconque des revendications précédentes, caractérisé en ce que le récepteur est un anticorps contre le ligand, le marqueur se trouvant sur le ligand est une enzyme, et le ligand marqué est présent selon une couverture inférieure à, égale à ou représentant un certain multiple de la quantité du ligand marqué utilisée quand l'analyse est effectuée par mise en contact de l'élément analytique simultanément avec un échantillon d'essai et un ligand marqué.

8. Elément selon la revendication 7, caractérisé en ce que le ligand marqué est présent au dessus de la couche d'étalement selon une couverture d'au moins 4 à 64 mg/m$^2$.

9. Elément selon la revendication 8, caractérisé en ce que le ligand comprend un haptène ou un analogue d'haptène, choisi parmi la digoxine, la phénytoïne, la carbamazépine, le phénobarbital, la théophylline et la thydroxine.

10. Elément selon la revendication 9, caractérisé en ce qu'il possède une couche additionnelle contenant un agent de transfert d'électrons et un tampon entre la couche de récepteur et le support.